(19) Europäisches Patentamt
European Patent Office
Office européen des brevets

(11) EP 3 171 805 B1

(12) EUROPEAN PATENT SPECIFICATION

(45) Date of publication and mention
of the grant of the patent:
**14.03.2018 Bulletin 2018/11**

(21) Application number: **15736506.5**

(22) Date of filing: **13.07.2015**

(51) Int Cl.:
**A61B 18/20** *(2006.01)*    **A61B 18/00** *(2006.01)*

(86) International application number:
**PCT/EP2015/065918**

(87) International publication number:
**WO 2016/012287 (28.01.2016 Gazette 2016/04)**

(54) **A DEVICE FOR CUTTING HAIR**

VORRICHTUNG ZUM SCHNEIDEN VON HAAR

DISPOSITIF POUR COUPE DE CHEVEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2014 EP 14178574**

(43) Date of publication of application:
**31.05.2017 Bulletin 2017/22**

(73) Proprietor: **Koninklijke Philips N.V.
5656 AE Eindhoven (NL)**

(72) Inventors:
• **MOESKOPS, Bastiaan Wilhelmus Maria
5656 AE Eindhoven (NL)**
• **JOHNSON, Mark Thomas
5656 AE Eindhoven (NL)**

• **COOMBS, James Howard
5656 AE Eindhoven (NL)**
• **VERHAGEN, Rieko
5656 AE Eindhoven (NL)**
• **CIUHU, Calina
5656 AE Eindhoven (NL)**
• **JUTTE, Petrus Theodorus
5656 AE Eidnhoven (NL)**

(74) Representative: **Uittenbroek, Arie Leendert
Philips Intellectual Property & Standards
High Tech Campus 5
5656 AE Eindhoven (NL)**

(56) References cited:
**WO-A1-92/16338    WO-A1-95/33600
GB-A- 2 495 248    US-A- 5 993 440**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a device for cutting hair using a laser beam. The present invention also relates to a method of cutting hair, and a computer program comprising instructions which, when executed by at least one processor, cause the method to be performed.

BACKGROUND OF THE INVENTION

It is known to use a laser beam to sever hair as an alternative to an arrangement of mechanical cutting blades. Hair exposed to a laser beam will absorb energy from the laser beam and the hair will either be severed by vaporisation or by laser induced optical breakdown and a resulting shockwave. A laser beam requires no moving parts and so the problem of cutting elements becoming worn or blunt is eliminated. Moreover, use of a laser beam to sever hair avoids skin irritation caused by the sharp edges of mechanical blades contacting the skin.

It is known from WO95/33600 to provide a device for cutting hair comprising a skin contacting face that is arranged to be placed against a surface of the skin of a user during use;

an optical system configured to direct a cutting laser beam across a cutting zone parallel to and spaced from said skin contacting face to cut hairs extending into the cutting zone;

a laser beam sensor configured to generate information indicative of one or more optical properties of the cutting laser beam, including information indicative of the position and/or orientation of the path of the cutting laser beam; and

a controller configured to adjust one or more characteristics of the optical system in dependence on the information generated by the laser beam sensor and to adjust one or more characteristics of the optical system in dependence on the information generated by the laser beam sensor.

Shaving performance is typically measured by two criteria - closeness of shave and irritation of the skin. The cutting height is the distance between the surface of the skin and the point at which hairs are cut. A good performing shaver should minimise the cutting height and therefore minimise the remaining hair length by cutting the hairs as close as possible to the skin. However, positioning a laser beam close to the skin may cause skin irritation if heat and energy from the laser is incident on the skin. It is therefore necessary to protect the skin from a laser beam to avoid causing damage or irritation to the skin. Furthermore, the position of the laser beam in the cutting zone may vary due to mechanical stress and/or environmental stress and therefore reduce reliability.

SUMMARY OF THE INVENTION

It is an object of the invention to provide a device for cutting hair using a laser beam and/or a method of cutting hair which substantially alleviates or overcomes the problems mentioned above.

According to the present invention, there is provided a device for cutting hair in which the laser beam sensor is configured to generate information indicative of the distance between the cutting laser beam and a reference position on the device.

With this arrangement it is possible to control operation of the optical system and therefore the device in dependence on determination of one or more optical properties of the cutting laser beam. Therefore, it is possible for the controller to adjust a characteristic of the optical system to minimise irritation of the skin to reduce the effect of the laser beam inadvertently being incident on a user's skin.

With this arrangement, it is also possible for the path of the laser beam to be accurately determined such that it is possible to determine the position of the cutting laser beam. Therefore, it is possible to determine information indicative of the path of the laser beam, and therefore indicative of the position relative to skin at the cutting zone. This means that a characteristic of the optical system may be changed to minimise irritation of the skin.

The device may further comprise a skin sensor configured to generate information indicative of the distance between skin at the cutting zone and a reference position. The controller may be configured adjust one or more characteristics of the optical system in dependence on the information generated by the laser beam sensor and the skin sensor.

With this arrangement it is possible to accurately determine the distance between the lower edge of the cutting laser beam and the skin at the cutting zone. Therefore, it is possible to determine when the gap between the cutting laser beam and the skin is below or above a certain level and so adjust a characteristic of the optical system accordingly.

The reference position on the device may be the skin contacting face.

Therefore, it is possible to easily determine the relative distances of the cutting laser beam and/or the skin at the cutting zone.

The laser beam sensor may be configured to generate information indicative of the intensity of the cutting laser beam.

With this arrangement it is possible to determine any reduction in the intensity of the laser beam along its optical path. Therefore, it is possible to determine whether any objects are incident with the cutting laser beam.

The controller may be configured to determine the presence of detritus along a path of the cutting laser beam based on the information indicative of the intensity of the cutting laser beam generated by the laser beam sensor.

With this arrangement it is possible to determine when the performance of the cutting laser beam is affected by the presence of detritus. Therefore, it is possible to adjust one or more characteristics of the optical system in dependence on the determination of detritus along the path of the cutting laser beam in an attempt to restore performance to a desired level.

The device may further comprise a skin property sensor configured to generate information indicative of one or more optical properties of skin at the cutting zone, and wherein the controller is configured to adjust one or more characteristics of the optical system in dependence on the information generated by the laser beam sensor and the skin property sensor. The skin property sensor may be configured to generate information indicative of the skin type.

Different skin types may be identified according to colour by use of the Fitzpatrick scale, in which skin type is identified in one of six types, between Type I which is identified as light, pale white and Type VI which is identified as black, very dark brown to black.

It has been determined that a device for cutting hair using a laser beam will, for a fixed laser parameter, give a different effect on skin of users having different skin colour. For example, it has been determined that the laser energy required to induce moderate skin damage, which is defined as local blistering, for Type II skin is around three times greater than the laser energy required to induce moderate skin damage for Type IV skin.

Therefore, by determining one or more optical qualities of the skin or the user it is possible to aid the minimization of any detrimental effects of the laser beam inadvertently being incident on a user's skin whilst maximising the shaving performance. Therefore, a uniform shaving performance can be maintained and variations in skin type can be accommodated by adjusting one or more characteristics of the optical system. Skin irritation may be reduced because operation of the cutting laser beam will be adjusted by one or more characteristics of the optical system in response to changes in one or more optical properties of the skin in the cutting zone.

The skin property sensor may be the skin sensor.

Therefore, the number of components is minimised and one sensor is able to provide information indicative of one or more optical properties of the skin and information indicative of the distance between skin at the cutting zone and the reference position.

One or more characteristics of the optical system that the controller may be configured to adjust may be the intensity of the cutting laser beam.

An advantage of this arrangement is that it is possible for feedback to be provided on the action of the cutting laser beam in the cutting zone. Furthermore, the energy imparted on the skin in the cutting zone may be controlled, and so minimise any irritation and/or damage. It may also be possible to reduce the effect, and therefore the irritation, potentially caused by the laser beam inadvertently being incident with the skin by changing the power or energy of the laser beam. This also means that it is possible to minimise the number of moving parts.

One or more characteristics of the optical system that the controller may be configured to adjust is the path of the cutting laser beam.

With this arrangement it is possible to adjust the path of the cutting laser beam to maximise the effectiveness of the device whilst minimising any irritation to skin in the cutting zone. This arrangement also allows any mechanical or environmental factors that may have an effect on the path of the cutting laser beam to be compensated for.

The controller may be configured to adjust the optical system to modify the distance between the path of the cutting laser beam and the skin contacting face.

Therefore, it is possible to adjust the spacing between the cutting laser beam and the skin contacting face to ensure that an optimal cutting action is performed by the device. With this arrangement it is possible to minimise the likelihood of the user's skin being incident with the cutting laser beam by adjusting the distance between the skin of a user and the cutting laser beam. Skin irritation will be reduced because the cutting laser beam may be moved relative to the skin in response to detected changes in the optical properties of the skin in the cutting zone and so the minimum distance between the cutting laser beam and the skin will change.

The controller may be configured to adjust the optical system of modify the lateral position of the path of the cutting laser beam across the cutting zone.

With this arrangement it is possible to adjust the lateral position of the cutting laser beam to compensate for any detritus that may be received in the cutting laser beam path in the cutting zone.

The optical system may comprise one or more optical components configured to act on the cutting laser beam, and an actuator configured to act on the one or more optical components, and wherein one or more characteristics of the optical system that the controller is configured to adjust may be operation of the actuator to modify the path of the cutting laser beam.

Movement of a component or components of the optical system helps to provide a simple and accurate adjustment of the position of the cutting laser beam in the cutting zone.

The optical component may be one or more optical lenses, one or more reflective elements, and/or the cutting laser beam generator.

Altering a reflection angle or position of a component can control the cutting height of the cutting laser beam without having to move a large assembly of components within the device. The actuator may be small, simple and lightweight.

The device may further comprise two or more actuators. The device may further comprise two or more laser beam sensors.

According to another aspect of the present invention, there is provided a method of cutting hair comprising operating an optical system to direct a cutting laser beam across a cutting zone parallel to and spaced from said skin contacting face to cut hairs extending into the cutting zone, generating information indicative of one or more optical properties of the cutting laser beam, generating information indicative of the distance between the cutting laser beam and a reference position using a laser beam sensor, and adjusting one or more characteristics of the optical system in dependence on the information generated by the laser beam sensor.

According to another aspect of the present invention, there is provided a computer program comprising instructions which, when executed by at least one processor, cause the method as described above to be performed.

According to the invention, there is also provided a device for cutting hair comprising a skin contacting face that is arranged to be placed against a surface of the skin of a user during use, an optical system configured to direct a cutting laser beam across a cutting zone parallel to and spaced from said skin contacting face to cut hairs extending into the cutting zone, a laser beam sensor configured to generate information indicative of one or more optical properties of the cutting laser beam and a controller configured to adjust one or more characteristics of the optical system in dependence on the information generated by the laser beam sensor. According to this aspect of the invention, the device may further comprise a skin property sensor configured to generate information indicative of one or more optical properties of skin at the cutting zone, and the controller may be configured to adjust one or more characteristics of the optical system in dependence on the information generated by the skin property sensor.

These and other aspects of the invention will be apparent from and elucidated with reference to the embodiments described hereinafter.

BRIEF DESCRIPTION OF THE DRAWINGS

Embodiments of the invention will now be described, by way of example only, with reference to the accompanying drawings, in which:

FIG. 1 shows a view of the cutting end of a device for cutting hair using a laser beam;
FIG. 2 shows a front view schematic diagram of the cutting end of the device of FIG. 1;
FIG. 3 shows a schematic block circuit diagram of the device of FIG. 2;
FIG. 4 shows a front view schematic diagram of a further embodiment of the cutting end of the device of FIG. 1;
FIG. 5 shows a front view schematic diagram of the device of FIG. 5;
FIG. 6 shows a schematic block circuit diagram of a different embodiment of the cutting end of the device of FIG. 6; and
FIG. 7 shows a plan view from above of a different embodiment of a cutting end of a device for cutting hair.

DETAILED DESCRIPTION OF EMBODIMENTS

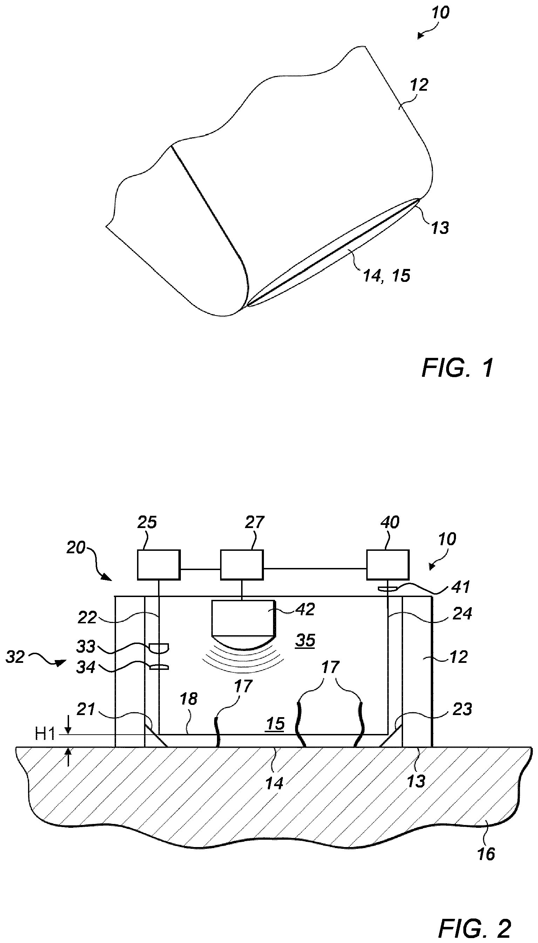

As shown in FIGS. 1 and 2, the device 10 for cutting hair comprises a main body 12 which includes a skin contacting face 13 having an opening 14. As shown in FIG. 2, during use, the skin contacting face 13 of the main body 12 is placed against skin 16 of a user and hairs 17 on the skin 16 protrude into the opening 14 of the main body 12 and into a cutting zone 15 defined therein. Within the main body 12, the device 10 comprises an optical system 20 which directs a cutting laser beam 18 across the cutting zone 15 so that the cutting laser beam 18 is parallel to and spaced from the skin contacting face 13. The cutting laser beam 18 also extends across the opening 14 of the main body 12. In this way, when the skin contacting face 13 of the main body 12 is placed against the skin 16 of the user, the cutting laser beam 18 is substantially parallel to and spaced from the surface of the skin 16 of the user and provides a substantially uniform cutting height across the cutting zone 15. The distance between the surface of the skin 16 and the cutting laser beam 18 in the cutting zone 15, represented as distance H1 in FIG. 2, is the cutting height.

The optical system 20 comprises a first reflective element 21 positioned on one side of the opening 14 and cutting zone 15. The first reflective element 21 is configured to reflect an incident section 22 of the cutting laser beam 18 across the opening 14 at the end of the device 10. That is, the first reflective element 21 is configured to reflect the incident section 22 of the cutting laser beam 18 across the cutting zone 15, such that the cutting laser beam 18 follows a path which is substantially parallel to and spaced from the skin contacting face 13 and opening 14 of the device 10.

On an opposite side of the cutting zone 15 to the first reflective element 21, a second reflective element 23 is positioned to reflect the cutting laser beam 18 away from the cutting zone 15, towards an energy dissipater (not shown) so that the 'used' section 24 of the cutting laser beam 18 is dissipated and can not interact with a part of the user or another part of the device. In the example shown in FIG. 2, the incident section 22 of the cutting laser beam 18 which is incident on the first reflective element 21 is perpendicular to the skin contacting face 13 of the main body 12 and the first reflective element 21 reflects the laser beam through 90 degrees such that it is parallel to the skin contacting face 13 of the main body 12. Similarly, the second reflective element 23 is configured to reflect the cutting laser beam 18 through 90 degrees and perpendicularly away from the cutting zone 15. However, it will be appreciated that the first and second reflective elements 21, 23 may be orientated differently or have different reflective angles, depending on the position and orientation of other parts of the optical system, such as a laser beam generator 25 and the energy dissipater (not shown). Furthermore, it will be appreciated that the first and second reflective elements 21, 23 may not be located at a side of the cutting zone 15. They may alternatively be located anywhere within the cutting zone 15, depending on the position, orientation and configuration of the other components of the optical system 20. The first and/or second reflective elements 21, 23 may be omitted. However, the cutting laser beam 18 within the cutting zone 15 should remain parallel to the skin contacting face 13 and opening 14 of the main body 12 so that the distance between the skin contacting face 13 and the cutting laser beam 18 is constant across the cutting zone 15. Any reflective elements described herein may comprise a mirror or a prism or any other optically reflective surface.

The optical system 20 of the device 10 further comprises the laser beam generator 25, such as a diode. As shown in FIG. 2, the optical system 20 includes a lens arrangement 32. The lens arrangement 32 comprises one or more lens elements. In the present embodiment, the lens arrangement 32 includes a collimating lens 33 and a focus lens 34. The laser beam emitted by the laser beam generator 25 is directed towards the collimating lens 33. The collimating lens 33 reduces or eliminates the divergence of the laser beam emitted by the laser beam generator 25. The collimated section or path of the laser beam is then focused by the focus lens 34 to cause the laser beam to converge.

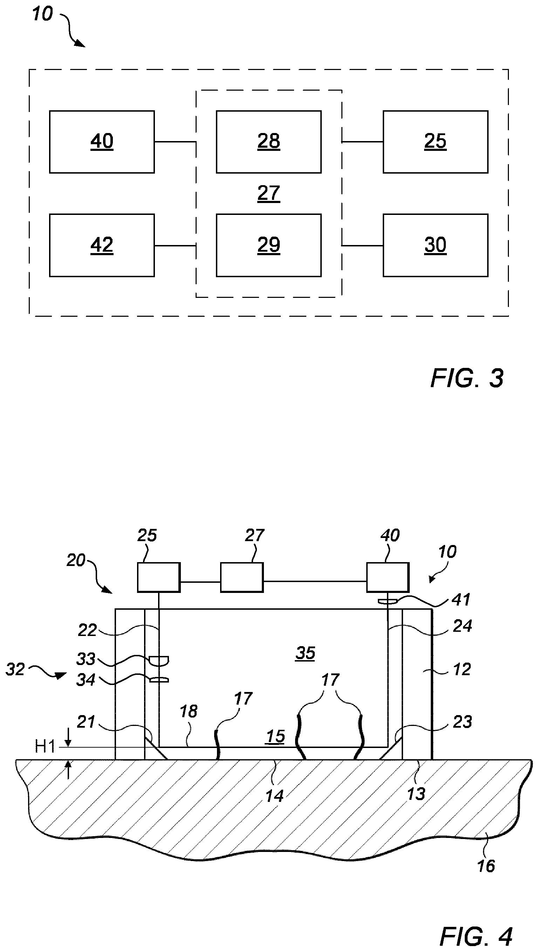

The device 10 also comprises a controller 27. The controller 27 is configured to control operation of the laser beam generator 25, and so control operation of the cutting laser beam 18. Referring to FIG. 3, the controller 27 comprises a processor 28 and a memory 29. The controller 27 is operable to operate the optical system 20.

The processor 28 may take any suitable form. For instance, the processor 28 may be or include a microcontroller, plural microcontrollers, circuitry, a single processor, or plural processors. The controller 27 may be formed of one or multiple modules.

The memory 29 takes any suitable form. The memory 29 may include a non-volatile memory and/or RAM. The non-volatile memory may include read only memory (ROM), a hard disk drive (HDD) or a solid state drive (SSD). The memory stores, amongst other things, an operating system. The memory may be disposed remotely. The RAM is used by the processor 28 for the temporary storage of data. The operating system may contain code which, when executed by the controller 27, controls operation of each of the hardware components of the device 10, or alternatively a system including the device 10. The controller 27 may be able to cause one or more objects, such as one or more profiles, to be stored remotely or locally by the memory 29. The controller 27 may be able to refer to one or more objects, such as one or more profiles, stored by the non-volatile memory and upload the one or more stored objects to the RAM.

The controller 27 is operable to operate the device 10 in response to an input, for example a user input 30. The controller 27 is configured to operate the optical system 20.

The user input 30 comprises some form of user interface. Optionally, the device 10 includes controls and/or displays for adjusting some operating characteristic of the device, such as the power. The user input 30 allows a user to operate the device 10, for example to turn the device 10 on and off. The user input 30 may, for example, be a button, touch screen or switch.

Other components that the device 10 may comprise, which are not shown, may include other optical components such as a filter or windows to limit the passage of detritus in the device 10. Other components necessary for the operation of the device may also be located within the main body 12, such as a battery or a connection to an external power cable (not shown). Moreover, the main body 12 of the device 10 may also comprise a handle and any switches, buttons or other controls and displays necessary to operate the device, which may form the user input 30.

The cutting zone 15 is formed in a chamber 35 in the main body 2. The opening 14 to the cutting zone 15 is defined by an opening to the chamber 35.

The device 10 is provided with a laser beam sensor 40. The laser beam sensor 40 is an electronic sensor which is configured to generate information indicative of one or more optical properties of the cutting laser beam 18 when the optical system 20 is operated. That information is provided to the controller 27. The controller 27 may then control operation of one or more components of the optical system 20 in dependence on the information provided by the laser beam sensor 40.

The laser beam sensor 40 is an optical imaging sensor. The optical imaging sensor may be a photodiode array. The laser beam sensor 40 is disposed at the end of the optical path of the cutting laser beam 18. In the present embodiment, the laser beam sensor 40 is at the energy dissipater (not shown). However, it will be understood that alternative sensor arrangements may be used.

The laser beam sensor 40 is configured to detect one or more optical properties of the cutting laser beam 18. A detector lens 41 is disposed on the cutting laser beam path prior to the laser beam sensor 40 to adjust the dimensions of the cutting laser beam 18 to suit the laser beam sensor 40. For example, the detector lens 41 may be configured to ensure that the dimensions of the cutting laser beam 18 at the laser beam sensor 40 correspond to the resolution of the laser beam sensor 40. The detector lens 41 may be omitted.

The laser beam sensor 40 intersects the path of the cutting laser beam 18. The laser beam sensor 40 is configured to determine one or more properties of the cutting laser beam, for example the position of the cutting laser beam 18, and/or the intensity of the cutting laser beam 18 at the laser beam sensor 40. When the laser beam sensor 40 is configured to provide information on the position of the cutting laser beam 18, the laser beam sensor 40 is configured to generate information on the position of the cutting laser beam 18 intersecting the laser beam sensor 40. When the laser beam sensor 40 is configured to provide information on the intensity of the cutting laser beam 18, the laser beam sensor 40 is configured to generate information on the intensity of the cutting laser beam 18 intersecting the laser beam sensor 40. In the present arrangement that is at the end of the laser beam path.

In the present embodiment, the laser beam sensor 40 is configured to generate information indicative of the position of the cutting laser beam 18. The laser beam sensor 40 is configured to generate information indicative of the lower edge of the cutting laser beam 18 in the cutting zone 15. The lower edge of the cutting laser beam 18 is the edge of the laser beam proximal to the skin contacting face 13. The lower edge of the cutting laser beam 18 in the present embodiment is defined as a periphery of the cutting laser beam 18 containing 99% of the laser intensity. However, it will be understood that an alternative definition for the lower edge of the cutting laser beam 18 may be used. The laser beam sensor 40 is therefore configured to provide information indicative of the distance between the lower edge of the cutting laser beam 18 and the reference position. In the present embodiment, the reference position is based on the skin contacting face 13. Therefore, the provided information indicative of the distance between the lower edge of the cutting laser beam 18 and the reference position is the perpendicular distance between the lower edge of the cutting laser beam 18 and the plane of the skin contacting face 13. However, it will be understood that an alternative reference position may be used. For example, the reference position may be a neutral calibrated position of the cutting laser beam 18.

The device 10 is provided with a skin sensor 42. The skin sensor 42 is an electronic sensor which is configured to generate information indicative of the distance between skin 16 at the cutting zone 15 and a reference position when the device 1 is positioned against the skin 16. This distance is the skin height. That information is provided to the controller 27 which controls operation of the laser generator 25. However, it will be understood that the skin sensor may be omitted. The skin sensor 42 is an optical sensor, such as a confocal lens which uses optical measuring techniques and does not need to contact the skin 16 to measure the distance between the surface of the skin 16 and the reference position on the device 10. The skin sensor 42 may be configured to generate information indicative of the position of the skin 16 by, for example, triangulation measurement, scattered light measurement and/or shadow measurement. In the present embodiment, the reference position is based on the skin contacting face 13. Therefore, the provided information indicative of the distance between the skin 16 at the cutting zone 15 and said reference position is the perpendicular distance between the skin at the cutting zone 15, as determined by the skin sensor 42, and the plane of the skin contacting face 13. However, it will be understood that an alternative reference position may be used. For example, the reference position may be a neutral calibrated position of the cutting laser beam 18.

The skin sensor 42 is disposed in the cutting zone 15. That is, in the present arrangement the skin sensor 42 is disposed in the chamber 35 in the main body 12. The skin sensor 42 is a non-contact skin distance sensor. That is, the skin sensor 42 is configured to measure the distance between skin 16 at the cutting zone 15 and the reference position when the device 1 is positioned against the skin 16. The skin sensor 42 may be a reflective or transmissive optical sensor. The skin sensor 42 may use one or more wavelengths of light in the visible and/or near infra-red radiation regions. However, it will be understood that alternative sensor arrangements may be used.

The skin sensor 42 comprises a light source (not shown) and a detector (not shown). A light path is defined between the light source and the detector. A user's skin forms part of the light path when the skin 42 is disposed at the opening 14 to the cutting zone 15. The skin sensor 42 is provided in the chamber 35. When the skin 16 of a user is disposed at the opening 14, the chamber 35 is enclosed.

Although in the present arrangement the skin sensor 42 is disposed in the cutting zone 15 and is configured to detect the skin 16 positioned at the opening 14 to the cutting zone 15, it will be understood that in an alternative embodiment the skin sensor 42 may be disposed outside the cutting zone 15. For example, the skin sensor 42 may be disposed to detect skin at a sensor opening (not shown) adjacent to the opening 14 to the cutting zone 15. An advantage of the skin sensor 42 being disposed in the cutting zone 15 is that it is more accurately able to determine the skin height at the cutting zone 15.

The controller 27 is configured to refer to the information generated by the laser beam sensor 40 indicative of the distance between the lower edge of the cutting laser beam 18 and the reference position. The controller 27 is configured to refer to the information generated by the skin sensor 42 indicative of the distance between skin at the cutting zone 15 and said reference position when the device 1 is positioned against the skin 16. The reference positions may be the same reference position, or may be different reference positions with a known spacing.

Based on the information generated by the laser beam sensor 40 and the skin sensor 42, the controller 27 is able to generate information indicative of the distance between the lower edge of the cutting laser beam 18 and the skin 16 at the cutting zone 15.

The controller 27 is configured to adjust the intensity of the cutting laser beam 18 in dependence on the determined distance between the lower edge of the cutting laser beam 18 and the skin 16 at the cutting zone 15. The distance between the lower edge of the cutting laser beam 18 and the skin 16 at the cutting zone 15 may vary in response to changes in the height of the skin 16 of a user within the cutting zone 15, for example due to skin height variations and the skin doming in the cutting zone 15. Therefore, the skin 16 may move closer to, and further away from, the cutting laser beam 18 during use of the device 20. The controller 27 is configured to reduce the intensity of the cutting laser beam 18, for example the power or the energy of the laser beam, when the distance between the lower edge of the cutting laser beam 18 and the skin 16 at the cutting zone 15 falls below a minimum threshold value. In this way, variations in the skin height, such as skin contours, can be accommodated whilst minimising skin irritation.

As the position of the cutting laser beam 18 relative to the reference position is determined, it will be appreciated that the controller 27 is able to compensate for any movement or discrepancies in the position and, therefore, path of the cutting laser beam 18 in the cutting zone 15. Due to the controller 27 being able to determine both the position of the lower edge of the cutting laser beam 18 in the cutting zone 15 and the skin 16 at the cutting zone 15, the controller 27 is able to accurately determine the distance between the lower edge of the cutting laser beam 18 and the skin 16. This also enables any variations during use or inbetween use to be taken into account. With such an arrangement, optical element alignment variations may be taken into account. Such optical element alignment variations, for example, may occur due to a user dropping the device 20.

Information indicative of the desired intensity for a given cutting height is stored in the memory 29 in a reference profile. The controller 27 is configured to refer to the reference profile of the desired intensity in response to information indicative of the distance between the lower edge of the cutting laser beam 18 and the skin 16 at the cutting zone 15. The reference profile may be stored in a look-up table. The reference profile may be stored by the memory 29. In such an arrangement, the controller 27 is configured to refer to the memory 29 to access the reference profile. In one embodiment, the reference profile is stored by the RAM. The reference profile provides information indicative of the desired intensity.

It will be understood that the controller 27 may be configured to select the reference profile from two or more reference profiles. That is, each reference profile may provide a desired operating intensity based on the determined cutting height. The two or more reference profiles may be stored in the memory 29. Alternatively, the reference profile may include two or more desired intensity levels from which the controller 27 is able to select based on the determined cutting height. The user may be able to select a desired closeness of shave. For example, in an embodiment in which the reference profile includes two or more desired intensity levels from which the controller 27 is able to select based on the determined cutting height, the user may be able to select from two or more reference profiles each of which has a different intensity level for a given cutting height.

When the device 10 is operated, the controller 27 is configured to operate the laser beam generator 25 to adjust the intensity of the cutting laser beam 18 in dependence on the cutting height determined by the controller 27 by reference to the information generated by the laser beam sensor 40 and the skin sensor 42.

In one embodiment, the controller 27 is operable to adjust the intensity of the cutting laser beam 18 in dependence on a minimum threshold value of the cutting height, as determined by the information generated by the laser beam sensor 40 and the skin sensor 42. In such an embodiment, the controller 27 is operable to operate the laser beam generator 25 so that the cutting laser beam 18 operates at a first intensity level when the cutting height is greater than the minimum threshold value, and the controller 27 is operable to operate the laser beam generator 25 so that the cutting laser beam 18 operates at a second intensity level when the cutting height is determined to be less than or equal to the minimum threshold value. The minimum threshold value is stored by the one or more reference profiles. With such an arrangement the controller 27 is configured to enact a step change in the intensity level of the cutting laser beam 18.

In an alternative embodiment, the reference profile may include one or more threshold values for the cutting height as determined by the controller 27 based on information provided by the laser beam sensor 40 and the skin sensor 42. In such an embodiment, each threshold value has a corresponding intensity level. The two or more threshold values and corresponding intensity levels may be stored by the reference profile. In another alternative, the controller 27 is configured to determine the desired intensity level for the cutting laser beam 18 by operating the laser beam generator 25 to constantly vary the intensity level based on information provided by the laser beam sensor 40 and the skin sensor 42.

With the above arrangements it is possible to ensure that the cutting laser beam 18 does not contact the skin of the user by actively monitoring the position of the cutting laser beam 18 with respect to the skin. Therefore, the controller 27 is able to compensate for any deterioration in the alignment of the cutting laser beam 18 during use, or between use, of the device 10. This helps to compensate for any mechanical wear, thermal expansion, build up of debris and/or accidental damage.

In one embodiment, the skin sensor 42 is also configured to generate information indicative of one or more optical properties of skin 16 at the cutting zone 15. Therefore, the skin sensor 42 is configured to act as a skin property sensor. In such an embodiment, the skin sensor 42, acting as a skin property sensor, is a non-contact skin colour sensor. That is, the skin sensor 42 is configured to measure the colour of the skin. The skin sensor 42 may use one or more wavelengths of light in the visible and/or near infra-red radiation regions to generate information indicative of the colour of the skin. The skin sensor 42 is configured to detect the optical absorption level of the skin, such that the skin melanin contributes to the absorption signal. However, it will be understood that alternative sensor arrangements may be used.

The controller 27 is configured to operate the laser beam generator 25 in dependence on the determined distance between the lower edge of the cutting laser beam 18 and the skin 16 together with the determined one or more optical properties of the skin 16.

The controller 27 is configured to determine both the position of the lower edge of the cutting laser beam 18 in the cutting zone 15 and the skin 16 at the cutting zone 15, based on information generated by the laser beam sensor 40 and the skin distance sensor 42. Therefore the controller 27 is able to accurately determine the distance between the lower edge of the cutting laser beam 18 and the skin 16. The controller 27 is also configured to determine information indicative of one or more optical properties of skin 16 at the cutting zone 15.

The controller 27 is configured to adjust the intensity of the cutting laser beam 18 in dependence on the determined distance between the lower edge of the cutting laser beam 18 and the skin 16 at the cutting zone 15, together with the determined one or more optical properties of the skin 16.

For example, in one embodiment the controller 27 is configured to select from two or more reference profiles in dependence on the information generated by the skin sensor 42 indicative of the colour of the skin. The controller 27 is configured to select between different reference profiles having different intensity level conditions for a given cutting height. For example, the controller 27 may be configured to operate the laser beam generator 25 to reduce the laser beam intensity by about a factor of three for a given cutting height when the skin type detected by the skin sensor 42 changes from a type II skin on the Fitzpatrick scale to a type IV skin on the Fitzpatrick scale. That is, the laser intensity set by the controller 27 for a given height and a lower skin type on the Fitzpatrick skin type scale is configured to be higher than the laser intensity set by the controller for a higher skin type on the Fitzpatrick skin type scale, and so the laser intensity is adjusted if a different skin type on the Fitzpatrick skin type scale is detected by the skin sensor 42.

The controller 27 may be configured to select the reference profile from two or more reference profiles. That is, each reference profile may provide a desired operating intensity based on the determined cutting height. The two or more reference profiles may be stored in the memory 29.

Although in the present embodiment the skin sensor 42 is configured to act as a skin distance sensor and a skin property sensor, it will be understood that in an alternative embodiment separate sensors are used as skin distance and skin property sensors.

Although in the above described embodiments, the controller 27 is configured to adjust the intensity of the cutting laser beam 18 in dependence on the information generated by the laser beam sensor 40 and the skin sensor 42 it will be understood that, in alternative embodiments of the device 10, the skin sensor 42 is omitted. Such an embodiment is shown in FIG. 4. The arrangement of this embodiment of the device 10 is generally the same as the embodiments described above, and so a detailed description will be omitted herein. Reference numerals will be retained, and refer to features and components described in detail above.

In embodiments in which the skin sensor 42 is omitted, the controller 27 is configured to refer to the laser beam sensor 40 to determine the cutting height, that is the distance between the lower edge of the cutting laser beam 18 and the skin 16. In such an embodiment the controller 27 is configured to determine any variation in the position of the cutting laser beam 18 relative to the reference position. The reference position may be a predetermined position of the cutting laser beam 18. Therefore, the controller 27 is able to recalibrate the device 10 in dependence on any variation in the position of the cutting laser beam 10 during and/or inbetween use.

In such an embodiment, the controller 27 is configured to adjust the intensity of the cutting laser beam 18 in dependence on the information generated by the laser beam sensor 40. The controller 27 is configured to refer to a reference profile to determine the desired intensity level of the cutting laser beam 18 in dependence on the information generated by the laser beam sensor 40 indicative of the position of the cutting laser beam 27.

It will also be understood that the embodiments described herein may also be used to maximise the efficiency of a device 10 which further comprises an arrangement to control the position of the skin, for example a guard (not shown). In such an arrangement, the position of the skin 16 relative to a predetermined path of the cutting laser beam 18 is restricted. However, the controller 27 is able to compensate for any misalignment of the cutting laser beam 18 by reference to the information indicative of the position and/or orientation of the path of the cutting laser beam as generated by the laser beam sensor 40. In such an embodiment, it will be understood that the skin sensor may be omitted.

In one embodiment, the device 10 has a skin sensor 42, but skin distance sensor capabilities are omitted. The controller 27 is configured to adjust the intensity of the cutting laser beam 18 in dependence on the information generated by the laser beam sensor 40 and information generated by the skin sensor 42 indicative of one or more optical properties of skin 16 at the cutting zone 15. In such an embodiment the controller 27 is not configured to adjust the intensity of the cutting laser beam 18 in dependence on information indicative of the distance between skin 16 at the cutting zone 15 and a reference position.

Although in the above described embodiments the one or more characteristics of the optical system that the controller 27 is configured to adjust is the intensity of the cutting laser beam 18, it will be understood that, in alternative embodiments, one or more other characteristics of the optical system may be adjusted. The one or more other characteristics of the optical system may be adjusted together adjustment of the intensity of the cutting laser beam 18, or as an alternative to adjustment of the intensity of the cutting laser beam 18.

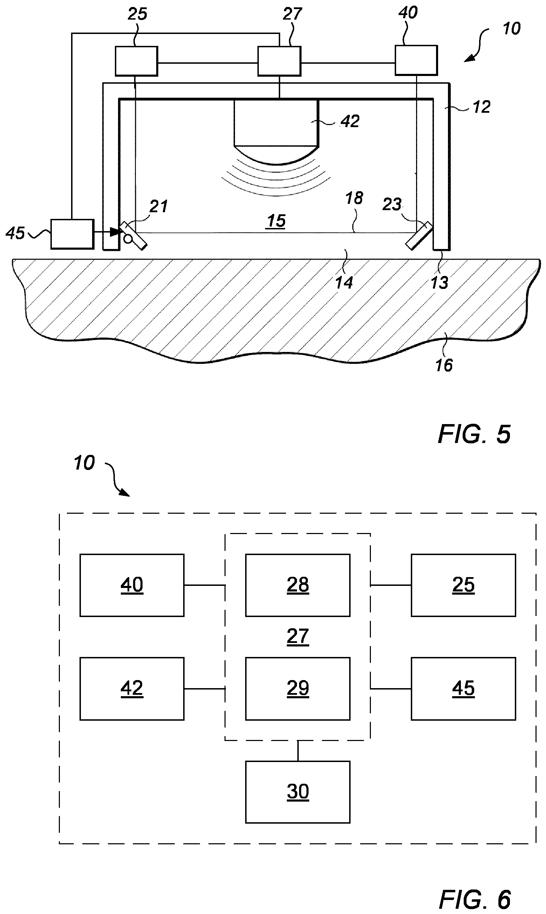

Referring now to FIGS. 5 and 6, another embodiment of the device 10 is shown. The arrangement of this embodiment of the device 10 as shown in FIGS. 5 and 6 is generally the same as the embodiments described above, and so a detailed description will be omitted herein. However, this embodiment of the device 10 further comprises an actuator 45 which is configured to act on one or more optical components of the optical system 20. Reference numerals will be retained and refer to features and components described in detail above.

In the embodiment of the device 10 shown in FIGS. 5 and 6, the actuator 45 is configured to act on the first reflective element 21. The actuator 45 is configured to cause the first reflective element 21 to move. The actuator 45 is arranged to cause rotation of the first reflective element 21. The first reflective element 21 is configured to rotate about a rotational axis which is perpendicular to the propagation direction of the incident section 22 of the cutting laser beam 18. The first reflective element 21 is positioned to reflect the incident section 22 of the cutting laser beam 18 across the cutting zone 15, as previously explained.

The actuator 45 is an electronic actuator, for example a voice coil actuator, spindle motor with gear or a piezo electrical translator. The actuator 45 acts on the first reflective element 21 in response to commands from the controller 27. The controller 27 is configured to operate the actuator 45 to control the propagation angle of the cutting laser beam 18 across the cutting zone 15. That is, movement of the first reflective element 21 allows the path of the cutting laser beam 18 across the cutting zone 15 to be adjusted, and therefore the cutting height.

The controller 27 is configured to operate the actuator 45 to move the first reflective element 21. Therefore, the path of the cutting laser beam 18 is varied in a direction perpendicular to the propagation direction of the cutting laser beam 18 in the cutting zone 15. The controller 27 is configured to refer to a reference profile to determine the desired position of the cutting laser beam 18 as determined by the controller 27 with reference to the information indicative of the position and/or orientation of the path of the cutting laser beam 18 generated by the laser beam sensor 40 and, optionally, information indicative of the position of the skin 16 and/or the skin type generated by the skin sensor 42. Therefore, the controller 27 is able to compensate for any misalignment of the optical system 20 during use or between uses.

In one embodiment, the reference profile relates to a desired position of the cutting laser beam 18, and the controller 27 is configured to refer to the reference profile and determine the variation from the desired position. In such an embodiment, the controller 27 is configured to adjust the orientation of the first reflective element 21 to restore the determined position of the cutting laser beam 18 in the cutting zone 15 to match the desired position as stored by the reference profile.

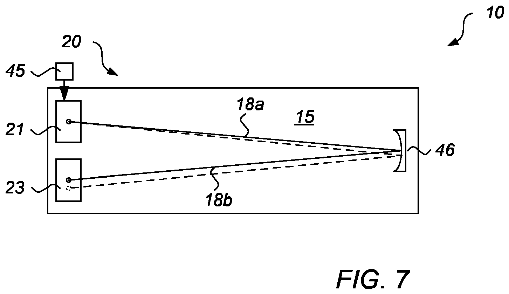

Referring now to FIG. 7, an alternative embodiment of the device 10 is shown. FIG. 7 shows a plan view from above of a different embodiment of the cutting end of the device for cutting hair. The arrangement of this embodiment of the device 10 as shown in FIG. 7 is generally the same as the embodiments described above, and so a detailed description will be omitted herein. However, in this embodiment of the device 10 the first and second reflective elements 21, 23 of the optical system 20 are disposed on one side of the cutting zone 15, and a third reflective element 46 is disposed on an opposing side to reflect a first part 18a of the cutting laser beam 18 from the first reflective element 21 back across the cutting zone 18 as a second part 18b of the cutting laser beam 18 to the second reflective element 23. Therefore, the third reflective element 46 forms an intermediate reflective element. Reference numerals will be retained and refer to features and components described in detail above.

In the present embodiment, the first reflective element 21 is configured to reflect the laser beam across the cutting zone 15. The second reflective element 23 is configured to reflect the laser beam away from the cutting zone 15. The first and second reflective elements 21, 23 act as described above with reference to the other embodiments, however, the third reflective element 46 is configured to reflect the cutting laser beam 18 back across the cutting zone 15. That is, the third reflective element 46 is configured to reflect the first part 18a of the cutting laser beam 18 so that the second part 18b extends across the cutting zone 18b along the same plane as the first part 18a. This creates a multibeam cutting action. This may be repeated multiple times through use of further intermediate reflective elements.

The third reflective element 46 in the present embodiment is a concave reflective element, however alternative arrangements are envisaged. The third reflective element 46 in the present embodiment is a refocusing mirror.

In this embodiment, the actuator 45 is configured to act on the first reflective element. The actuator 45 is configured to cause the first reflective element 21 to move. The actuator 45 is arranged to cause rotation of the first reflective element 21 about a rotational axis which is parallel to the propagation direction of the incident section 22 of the cutting laser beam 18. The first reflective element 21 is positioned to reflect the incident section 22 of the cutting laser beam 18 across the cutting zone 15, as previously explained. Therefore, the actuator 45 is configured to act on the first reflective element 21 to cause an adjustment of the lateral position of the cutting laser beam 18 across the cutting zone 15.

The actuator 45 is an electronic actuator, for example a voice coil actuator, spindle motor with gear or a piezo electrical translator. The actuator 45 acts on the first reflective element 21 in response to commands from the controller 27. The controller 27 is configured to operate the actuator 45 to control the lateral position of the first part 18a of the cutting laser beam 18 across the cutting zone 15. That is, the controller 27 is configured to adjust the path of the cutting laser beam parallel to the plane of the skin contacting face 13.

The controller 27 is configured to operate the actuator 45 to move the first reflective element 21. Therefore, the path of the cutting laser beam 18 is varied in a direction parallel to the plane of the skin contacting face 13.

In the present embodiment, the controller 27 is configured to refer to information generated by the laser beam sensor 40 indicative of the position of the cutting laser beam 18 in the cutting zone 15. The controller 27 is configured to operate the actuator 40 to adjust the position of the cutting laser beam 18 in dependence on the feedback generated by the laser beam sensor 40.

The controller 27 is configured to refer to a reference profile to determine the desired position of the cutting laser beam 18 as determined by the controller with reference to the information indicative of the position and/or orientation of the path of the cutting laser beam 18 generated by the laser beam sensor 40 and, optionally, information indicative of the position of the skin 16 and/or the skin type generated by the skin sensor 42. Therefore, the controller 27 is able to compensate for any misalignment of the optical system 20 during use or between uses.

In one embodiment, the reference profile relates to a desired position of the cutting laser beam 18, and the controller 27 is configured to refer to the reference profile and determine the variation from the desired position. In such an embodiment, the controller 27 is configured to adjust the orientation of the first reflective element 21 to restore the determined position of the cutting laser beam 18 in the cutting zone 15 to match the desired position as stored by the reference profile.

In one embodiment, the controller 27 is configured to operate the actuator 45 in dependency on information indicative of the intensity level of the cutting laser beam 18 at the laser beam sensor 40 as generated by the laser beam sensor 40. When the device 10 is operated, the controller 27 is configured to refer to information generated by the laser beam sensor 40. The controller 27 is configured to refer to information indicative of the intensity of the cutting laser beam 18 at the laser beam sensor 40.

It will be understood that, during use, debris may accumulate in the cutting zone 15. Therefore, debris may collate along the path of the cutting laser beam 18, and interfere with the cutting laser beam 18. For example, detritus may accumulate on the third reflective element 46 on the path of the cutting laser beam 18. Therefore, the intensity of the cutting laser beam 18 along the second part 18b of the cutting laser beam may be reduced in view of the interaction with the detritus. The controller 27 will be able to determine that detritus is disposed along the path of the cutting laser beam 18 in response to the information indicative of the intensity of the cutting laser beam 18 at the laser beam sensor 40.

The controller 27 may refer to a reference profile indicative of the desired intensity. The controller 27 is configured to operate the actuator 45 to act on the first reflective element 21 to adjust the path of the cutting laser beam 18. Movement of the path of the first part 18a of the cutting laser beam 18 will cause the cutting laser beam 18 to intersect with a different portion of the third reflective element 46. Therefore, the first part 18a of the cutting laser beam 18 may be directed to a portion of the third reflective element 46 which is clear of detritus. The controller 27 is configured to determine when the path of the cutting laser beam 18 is adjusted to be clear of detritus in response to the information indicative of the intensity of the cutting laser beam 18 at the laser beam sensor 40. That is, the intensity will return to a predefined level.

Although in the present embodiment, the actuator 45 is configured to act on the first reflective element 21, it will be understood that the actuator 45 may act on another optical component to adjust the path of the cutting laser beam 18 based on information indicative of the intensity of the cutting laser beam 18 at the laser beam sensor 40.

With the embodiment described above, it is possible for the controller 27 to adjust the position of the cutting laser beam 18 in the cutting zone 15 parallel to the skin contacting face 13, whilst maintaining the cutting height. Therefore, the shaving closeness and skin irritation level of the device 10 may be maintained, whilst ensuring that the intensity of the cutting laser beam 18 in the cutting zone 15 is maintained at a consistent level. This means that the efficiency of the device 10 may be maintained. However, it will also be understood that the controller 27 may be configured to adjust one or more other operating characteristics of the optical system 20.

In the embodiments described above in which the actuator 45 is configured to act on one or more of the optical components of the optical system 20, the movement that the actuator 45 is configured to cause is rotation. In particular, in the embodiments described herein with reference to FIGS. 5 and 6, the actuator 45 is configured to act on the first reflective element 21 to cause rotation of the first reflective element 21. However, it will be understood that alternative arrangements are envisaged.

In embodiments, it will be understood that the actuator 45 may be configured to act on one or more of the optical components of the optical system 20 to cause translation of the one or more optical components of the optical system 20. For example, in one embodiment the actuator 45 is configured to act on the first reflective element 21 to cause a translational movement of the first reflective element 21. The translational movement may be in a direction towards and/or away from the laser beam generator 25; that is perpendicular to the propagation direction of the cutting laser beam 18 across the cutting zone 15. It will be understood that the second reflective element 23 may move together with the first reflective element 21, or be dimensioned to allow for movement of the first reflective element 21.

In an alternative embodiment, the translational movement of the first reflective element 21 may be in a direction towards and away from the second reflective element 23; that is parallel to the path of the cutting laser beam 18 across the cutting zone 15.

The actuator 45 may alternatively, or also, act on the laser beam generator 25 to cause translation of the laser beam generator 25 itself. In such an embodiment, the actuator 45 is configured to act on the laser beam generator 25 to cause a translational movement parallel to the propagation direction of the cutting laser beam 18 across the cutting zone 15.

The actuator 45 may alternatively, or also, act on the lens arrangement 32. For example, the actuator 45 may act on the focus lens 34. The actuator may cause a translational movement of the focus lens 34 in a direction perpendicular to the optical axis of the cutting laser beam 18 through the lens arrangement 32. For example, if the decentering of the collimating lens 33 with respect to the laser is defined as $\Delta$, the required translation, d, of the cylindrical focus lens for compensation is given by:

$$d = -\frac{f_y}{f_c}\Delta$$

wherein $f_c$ is the focal length of the collimating lens 33, and $f_y$ is the focal length of the cylindrical focus lens.

In each of the above embodiments, the one or more characteristics of the optical system 20 that are adjusted in dependence on one or more of the optical properties of the cutting laser beam 18 detected by the laser beam sensor 40 may be adjusted dynamically as the device 10 is moved over the skin 16 of a user, dependent on the specific generated information indicative of the distance between the lower edge of the cutting laser beam 18 and the skin 16. An advantage of this arrangement is that the device is able to dynamically adjust operation of the cutting laser beam 18 to each part of a user's skin. Alternatively, the controller 27 may be configured to determine the optical properties of the skin 16 upon the device 10 being placed against the skin 16, or at a predetermined stage. An advantage of this arrangement is that a consistent operation of the device 10 is obtained.

Although in the above described embodiments including the skin sensor, the skin sensor is configured to generate information indicative of the distance between the skin at the cutting zone and a reference position, and that the controller is configured to refer to the information generated by the skin sensor to generate information indicative of the distance between the skin at the cutting zone and the lower edge of the cutting laser beam, it will be understood that in an alternative embodiment the controller is configured to generate information indicative of the distance between the skin at the cutting zone and the lower edge of the cutting laser beam based solely on the information provided by the laser beam sensor. However, the skin sensor may still be configured to generate information indicative of one or more optical properties of skin 16 at the cutting zone 15. That is, the skin sensor may be a skin property sensor, but not a skin distance sensor. In such an embodiment, the controller may be configured to adjust one or more characteristics of the optical system, for example the intensity of the cutting laser beam, in dependence on the information generated by the laser beam sensor together with information provided by the skin sensor acting as a skin property sensor.

Although in the above described embodiments, adjustment of the or each optical component is achieved through adjustment of the orientation of the or each optical component, it will be understood that in an alternative embodiment adjustment of the or each optical component is achieved by having interchangeable optical components. In one embodiment, the or each actuator is operable to interchange two or more optical components.

Although in the above described arrangements, the laser beam sensor is disposed proximate to the end of the cutting laser beam path, it will be understood that alternative arrangements are possible. For example, the laser beam sensor may be disposed at an optical component, for example at a reflective element. Furthermore, in embodiments two or more laser beam sensors are disposed along the cutting laser beam path, spaced from each other. This allows for a more robust and effective detection of the cutting laser beam. For example, two or more laser beam sensors may be disposed in multiple beam reflection paths to determine properties of the cutting laser beam in each section of the cutting laser beam path.

It will be appreciated that the term "comprising" does not exclude other elements or steps and that the indefinite article "a" or "an" does not exclude a plurality. The mere fact that certain measures are recited in mutually different dependent claims does not indicate that a combination of these measures cannot be used to an advantage. Any reference signs in the claims should not be construed as limiting the scope of the claims.

Although claims have been formulated in this application to particular combinations of features, it should be understood that the scope of the disclosure of the present invention also includes any novel features or any novel combinations of features disclosed herein either explicitly or implicitly or any generalisation thereof, whether or not it relates to the same invention as presently claimed in any claim and whether or not it mitigates any or all of the same technical problems as does the parent invention. The applicants hereby give notice that new claims may be formulated to such features and/or combinations of features during the prosecution of the present application or of any further application derived therefrom.

**Claims**

1. A device (10) for cutting hair comprising
a skin contacting face (13) that is arranged to be placed against a surface of the skin of a user during use;
an optical system (20) configured to direct a cutting laser beam (18) across a cutting zone (15) parallel to and spaced from said skin contacting face to cut hairs extending into the cutting zone;
a laser beam sensor (40) configured to generate information indicative of one or more optical properties of the cutting laser beam (18), including information indicative of the position and/or orientation of the path of the cutting laser beam (18); and
a controller (27) configured to adjust one or more characteristics of the optical system (20) in dependence on the information generated by the laser beam sensor (40);
**characterised in that** that the laser beam sensor (40) is configured to generate information indicative of the distance between the cutting laser beam (18) and a reference position on the device.

2. The device according to claim 1, further comprising a skin sensor (42) configured to generate information indicative of the distance between skin at the cutting zone (15) and the reference position, and wherein the controller (27) is configured to adjust one or more characteristics of the optical system (20) in dependence on the information generated by the laser beam sensor (40) and the skin sensor (42).

3. The device according to claim 1 or 2, wherein the reference position on the device is the skin contacting face (13).

4. The device according to any one of the preceding claims, wherein the laser beam sensor (40) is configured to generate information indicative of the intensity of the cutting laser beam (18).

5. The device according to claim 4, wherein the controller (27) is configured to determine the presence of detritus along a path of the cutting laser beam (18) based on the information indicative of the intensity of the cutting laser beam generated by the laser beam sensor (40).

6. The device according to any one of the preceding claims, further comprising a skin property sensor (42) configured to generate information indicative of one or more optical properties of skin at the cutting zone (15), and wherein the controller (27) is configured to adjust one or more characteristics of the optical system (20) in dependence on the information generated by the skin property sensor.

7. The device according to any one of the preceding claims, wherein one or more characteristics of the optical system (20) that the controller (40) is configured to adjust is the intensity of the cutting laser beam (18).

8. The device according to any one of the preceding claims, wherein one or more characteristics of the optical system (20) that the controller (40) is configured to adjust is the path of the cutting laser beam (18).

9. The device according to claim 8, wherein the controller (40) is configured to adjust the optical system (20) to modify the distance between the path of the cutting laser beam (18) and the skin contacting face (13).

10. The device according to claim 8 or claim 9, wherein the controller (40) is configured to adjust the optical system (20) to modify the lateral position of the path of the cutting laser beam (18) across the cutting zone (15).

11. The device according to claim 10, wherein the optical system (20) comprises one or more optical components (33,34,21) configured to act on the cutting laser beam (18), and an actuator (45) configured to act on the one or more optical components, and wherein one or more characteristics of the optical system that the controller (40) is configured to adjust is operation of the actuator to modify the path of the cutting laser beam.

12. A method of cutting hair comprising
operating an optical system (20) to direct a cutting laser beam (18) across a cutting zone (15) parallel to and spaced from said skin contacting face (13) to cut hairs extending into the cutting zone,
generating information indicative of one or more optical properties of the cutting laser beam (18),
generating information indicative of the distance between the cutting laser beam (18) and a reference position using a laser beam sensor (40), and
adjusting one or more characteristics of the optical system in dependence on the information generated by the laser beam sensor (40).

13. A computer program comprising instructions which, when executed by at least one processor, cause the method of claim 12 to be performed.

**Patentansprüche**

1. Vorrichtung (10) zum Schneiden von Haar, umfassend

   eine Hautkontaktfläche (13), die angeordnet ist, um gegen eine Hautoberfläche eines Benutzers beim Gebrauch gehalten zu werden;

   ein optisches System (20), das konfiguriert ist, um einen Schneidlaserstrahl (18) über einen Schneidbereich (15) parallel zu und beabstandet von der Hautkontaktfläche zu leiten, um Haare zu schneiden, die sich in den Schneidbereich erstrecken;

   einen Laserstrahlsensor (40), der konfiguriert ist, um Informationen zu erzeugen, die bezeichnend sind für eine oder mehrere optische Eigenschaften des Schneidlaserstrahls (18), einschließlich Informationen, die bezeichnend sind für die Position und/oder Ausrichtung des Weges des Schneidlaserstrahls (18); und

   eine Steuerung (27), die konfiguriert ist, um ein oder mehrere Merkmale des optischen Systems (20) in Abhängigkeit von den Informationen, die von dem Laserstrahlsensor (40) erzeugt werden, anzupassen;

   **dadurch gekennzeichnet, dass** der Laserstrahlsensor (40) konfiguriert ist, um Informationen zu erzeugen, die bezeichnend sind für den Abstand zwischen dem Schneidlaserstrahl (18) und einer Referenzposition auf der Vorrichtung.

2. Vorrichtung nach Anspruch 1, weiter umfassend einen Hautsensor (42), der konfiguriert ist, um Informationen zu erzeugen, die bezeichnend sind für den Abstand zwischen Haut am Schneidbereich (15) und der Referenzposition, und wobei die Steuerung (27) konfiguriert ist, um ein oder mehrere Merkmale des optischen Systems (20) in Abhängigkeit von den Informationen, die von dem Laserstrahlsensor (40) und dem Hautsensor (42) erzeugt werden, anzupassen.

3. Vorrichtung nach Anspruch 1 oder 2, wobei die Referenzposition auf der Vorrichtung die Hautkontaktfläche (13) ist.

4. Vorrichtung nach einem der vorstehenden Ansprüche, wobei der Laserstrahlsensor (40) konfiguriert ist, um Informationen zu erzeugen, die bezeichnend sind für die Stärke des Schneidlaserstrahls (18).

5. Vorrichtung nach Anspruch 4, wobei die Steuerung (27) konfiguriert ist, um das Vorhandensein von Rückständen entlang eines Weges des Schneidlaserstrahls (18) basierend auf den Informationen zu bestimmen, die bezeichnend sind für die Stärke des Schneidlaserstrahls, die von dem Laserstrahlsensor (40) erzeugt werden.

6. Vorrichtung nach einem der vorstehenden Ansprüche, weiter umfassend einen Hauteigenschaftssensor (42), der konfiguriert ist, um Informationen zu erzeugen, die bezeichnend sind für eine oder mehrere optische Hauteigenschaften am Schneidbereich (15), und wobei die Steuerung (27) konfiguriert ist, um ein oder mehrere Merkmale des optischen Systems (20) in Abhängigkeit von den Informationen, die von dem Hauteigenschaftssensor erzeugt werden, anzupassen.

7. Vorrichtung nach einem der vorstehenden Ansprüche, wobei ein oder mehrere Merkmale des optischen Systems (20), zu deren Anpassung die Steuerung (40) konfiguriert ist, die Stärke des Schneidlaserstrahls (18) ist.

8. Vorrichtung nach einem der vorstehenden Ansprüche, wobei ein oder mehrere Merkmale des optischen Systems (20), zu deren Anpassung die Steuerung (40) konfiguriert ist, der Weg des Schneidlaserstrahls (18) ist.

9. Vorrichtung nach Anspruch 8, wobei die Steuerung (40) konfiguriert ist, um das optische System (20) anzupassen, um den Abstand zwischen dem Weg des Schneidlaserstrahls (18) und der Hautkontaktfläche (13) zu ändern.

10. Vorrichtung nach Anspruch 8 oder Anspruch 9, wobei die Steuerung (40) konfiguriert ist, um das optische System (20) anzupassen, um die seitliche Position des Weges des Schneidlaserstrahls (18) über den Schneidbereich (15) zu ändern.

11. Vorrichtung nach Anspruch 10, wobei das optische System (20) eine oder mehrere optische Komponenten (33, 34, 21) umfasst, die konfiguriert sind, um auf den Schneidlaserstrahl (18) zu wirken, und ein Betätigungselement (45), das konfiguriert ist, um auf die eine oder mehreren optischen Komponenten zu wirken, und wobei ein oder mehrere Merkmale des optischen Systems, zu deren Anpassung die Steuerung (40) konfiguriert ist, die Bedienung des Betätigungselements ist, um den Weg des Schneidlaserstrahls zu ändern.

12. Verfahren zum Schneiden von Haar, umfassend Bedienen eines optischen Systems (20), um einen Schneidlaserstrahl (18) über einen Schneidbereich (15) parallel zu und beabstandet von der Hautkontaktfläche (13) zu leiten, um Haare zu schneiden, die sich in den Schneidbereich erstrecken,

    Erzeugen von Informationen, die bezeichnend sind für eine oder mehrere optische Eigenschaften des Schneidlaserstrahls (18),

    Erzeugen von Informationen, die bezeichnend sind für den Abstand zwischen dem Schneidlaserstrahl (18) und einer Referenzposition unter Verwendung eines Laserstrahlsensors (40), und

Anpassen eines oder mehrerer Merkmale des optischen Systems in Abhängigkeit von den Informationen, die von dem Laserstrahlsensor (40) erzeugt werden.

13. Computerprogramm, umfassend Anweisungen, die bei Ausführung durch mindestens einen Prozessor die Durchführung des Verfahrens nach Anspruch 12 veranlassen.

**Revendications**

1. Dispositif (10) permettant de couper des poils comprenant
   une face en contact avec la peau (13) qui est agencée pour être placée contre une surface de la peau d'un utilisateur pendant l'utilisation ;
   un système optique (20) configuré pour diriger un faisceau laser de coupe (18) à travers une zone de coupe (15) parallèle à et espacée de ladite face en contact avec la peau pour couper des poils s'étendant dans la zone de coupe ;
   un capteur de faisceau laser (40) configuré pour générer des informations indiquant une ou plusieurs propriétés optiques du faisceau laser de coupe (18), y compris des informations indiquant la position et/ou l'orientation du trajet du faisceau laser de coupe (18) ; et
   un dispositif de commande (27) configuré pour ajuster une ou plusieurs caractéristiques du système optique (20) en fonction des informations générées par le capteur de faisceau laser (40) ;
   **caractérisé en ce que** le capteur de faisceau laser (40) est configuré pour générer des informations indiquant la distance entre le faisceau laser de coupe (18) et une position de référence sur le dispositif.

2. Dispositif selon la revendication 1, comprenant en outre un capteur de peau (42) configuré pour générer des informations indiquant la distance entre la peau au niveau de la zone de coupe (15) et la position de référence, et dans lequel le dispositif de commande (27) est configuré pour ajuster une ou plusieurs caractéristiques du système optique (20) en fonction des informations générées par le capteur de faisceau laser (40) et le capteur de peau (42).

3. Dispositif selon la revendication 1 ou 2, dans lequel la position de référence sur le dispositif est la face en contact avec la peau (13).

4. Dispositif selon l'une quelconque des revendications précédentes, dans lequel le capteur de faisceau laser (40) est configuré pour générer des informations indiquant l'intensité du faisceau laser de coupe (18).

5. Dispositif selon la revendication 4, dans lequel le dispositif de commande (27) est configuré pour déterminer la présence de détritus le long d'un trajet du faisceau laser de coupe (18) sur la base des informations indiquant l'intensité du faisceau laser de coupe généré par le capteur de faisceau laser (40).

6. Dispositif selon l'une quelconque des revendications précédentes, comprenant en outre un capteur de propriétés de la peau (42) configuré pour générer des informations indiquant une ou plusieurs propriétés optiques de la peau au niveau de la zone de coupe (15), et dans lequel le dispositif de commande (27) est configuré pour ajuster une ou plusieurs caractéristiques du système optique (20) en fonction des informations générées par le capteur de propriétés de la peau.

7. Dispositif selon l'une quelconque des revendications précédentes, dans lequel une ou plusieurs caractéristiques du système optique (20) que le dispositif de commande (40) est configuré pour ajuster est l'intensité du faisceau laser de coupe (18).

8. Dispositif selon l'une quelconque des revendications précédentes, dans lequel une ou plusieurs caractéristiques du système optique (20) que le dispositif de commande (40) est configuré pour ajuster est le trajet du faisceau laser de coupe (18).

9. Dispositif selon la revendication 8, dans lequel le dispositif de commande (40) est configuré pour ajuster le système optique (20) pour modifier la distance entre le trajet du faisceau laser de coupe (18) et la face en contact avec la peau (13).

10. Dispositif selon la revendication 8 ou la revendication 9, dans lequel le dispositif de commande (40) est configuré pour ajuster le système optique (20) pour modifier la position latérale du trajet du faisceau laser de coupe (18) à travers la zone de coupe (15).

11. Dispositif selon la revendication 10, dans lequel le système optique (20) comprend un ou plusieurs composants optiques (33, 34, 21) configurés pour agir sur le faisceau laser de coupe (18), et un actionneur (45) configuré pour agir sur les un ou plusieurs composants optiques, et dans lequel une ou plusieurs caractéristiques du système optique que le dispositif de commande (40) est configuré pour ajuster est le fonctionnement de l'actionneur pour modifier le trajet du faisceau laser de coupe.

12. Procédé de coupe de poils comprenant
    le fonctionnement d'un système optique (20) pour diriger un faisceau laser de coupe (18) à travers une zone de coupe (15) parallèle à et espacée de ladite face en contact avec la peau (13) pour couper des poils s'étendant dans la zone de coupe,

la génération d'informations indiquant une ou plusieurs propriétés optiques du faisceau laser de coupe (18),

la génération d'informations indiquant la distance entre le faisceau laser de coupe (18) et une position de référence à l'aide d'un capteur de faisceau laser (40), et

l'ajustement d'une ou plusieurs caractéristiques du système optique en fonction des informations générées par le capteur de faisceau laser (40).

13. Programme informatique comprenant des instructions qui, lorsqu'elles sont exécutées par au moins un processeur, amènent le procédé de la revendication 12 à être mis en oeuvre.

10

12

13

14, 15

*FIG. 1*

20

25    27    40

10

41

22    24

42

35

12

33

34

32

17

17

18    17    23

21

15

H1

14    13

16

*FIG. 2*

10

| 40 | | 28 | | 25 |

27

| 42 | | 29 | | 30 |

*FIG. 3*

*FIG. 4*

EP 3 171 805 B1

FIG. 5

FIG. 6

*FIG. 7*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9533600 A